# EUROPÄISCHE PATENTSCHRIFT

(11) **EP 1 137 430 B1**
(45) Veröffentlichungstag und Bekanntmachung des Hinweises auf die Patenterteilung: **12.03.2003**
(21) Anmeldenummer: 99964547.6
(22) Anmeldetag: 10.12.1999
(51) Int. Cl.: A61K 38/16, A61P 11/00

(54) **THERAPEUTIKUM ZUR UNTERDRÜCKUNG VON SCHNARCHGERÄUSCHEN**
THERAPEUTIC AGENT FOR THE SUPPRESSION OF SNORING NOISES
MEDICAMENT ANTI-RONFLEMENTS

(30) Priorität: 10.12.1998 DE 19856897
(43) Veröffentlichungstag der Anmeldung: 04.10.2001
(73) Patentinhaber: BioteCon Gesellschaft für Biotechnologische Entwicklung und Consulting mbH, 10589 Berlin (DE)
(72) Erfinder: BIGALKE, Hans, D-30419 Hannover (DE); FREVERT, Jürgen, D-10625 Berlin (DE)
(74) Vertreter: Dehmel, Albrecht, Dr.
(86) Internationale Anmeldenummer: EP9909791
(87) Internationale Veröffentlichungsnummer: WO00033863

(56) Entgegenhaltungen:
- WO-A-94/00481
- WO-A-94/28923
- US-A- 4 551 473
- US-A- 5 512 547
- US-A- 5 721 215
- HAMBLETON P: "CLOSTRIDIUM BOTULINUM TOXINS: A GENERAL REVIEW OF INVOLVEMENT IN DISEASE, STRUCTURE, MODE OF ACTION AND PREPARATION FOR CLINICAL USE" JOURNAL OF NEUROLOGY - ZEITSCHRIFT FUER NEUROLOGIE,DE,SPRINGER VERLAG, BERLIN, Bd. 239, Nr. 1, 1992, Seiten 16-20, XP002009346 ISSN: 0340-5354
- HENESON N: "DEADLY TOXIN CALMS EXCITED MUSCLES" NEW SCIENTIST,GB,NEW SCIENCE PUBLICATIONS, LONDON, Nr. 1746, 8. Dezember 1990 (1990-12-08), Seite 24 XP002025654 ISSN: 0028-6664

## Beschreibung

### Hintergrund der Erfindung:

Nächtliches Schnarchen ist nicht nur ein psychosoziales Problem. Dieses Leiden stellt einen Risikofaktor für Erkrankungen des kardiovasculären Systems dar, wie Hypertonie (Kleitmann 1963; Lugaresi et al. 1983; Hoffstein et al. 1991) und myocardiale (Waller und Bhopal 1989; Koskenvuo et al. 1985) sowie zentrale Ischämie (Koskenvuo et al. 1987). Weiterhin sind Patienten mit verengten Atemwegen besonders bedroht, eine Schlafapnoe zu entwickeln, die mit einer erhöhten Mortalität einhergeht (He et al. 1988; Hoch et al. 1986).

Der weiche Gaumen setzt sich aus den quergestreiften Muskeln M. tensor veli palatini, M. pterygoideus, M. genioglossus, M. geniohyoideus und M. sternohyoideus zusammen. Während der Einatmung werden einige dieser Muskel aktiviert. Der Grad der Aktivierung hängt weiterhin von einigen anderen Faktoren ab, die im Einzelnen noch nicht geklärt sind. Eine Störung des Zusammenspiels der Muskeln kann Schnarchen auslösen. Eine Ursache des Schnarchens ist ein erhöhter Tonus des M. tensor veli palatini während der Tiefschlafphase. In der REM-Phase sinkt der Muskeltonus, und das Schnarchen nimmt ab bzw. hört auf (Lugaresi et al. 1994). Wird jedoch die Anspannung der Muskulatur erhöht, entsteht ein Resonanzboden, der durch die vorbeistreichende Atmungsluft in Schwingungen gerät. Liegt die Schwingfrequenz. Über 20 - 30 Hz, wird sie hörbar. Je höher die Frequenz der Schwingung ist, desto höher wird die Tonlage des Schnarchens. Die Frequenz hängt von der Anspannung des Muskels ab: ein stärker angespannter Muskel schwingt mit einer höheren Frequenz als ein leicht angespannter, ein erschlaffter Muskel schwingt nicht. Die Lautstärke des Schnarchens korreliert mit der Schwingungsamplitude, die von der Geschwindigkeit der vorbeistreichenden Atemluft bestimmt wird.

Werden die pathologisch tonisierten, quergestreiften Muskel mit einem langwirksamen Muskelrelaxants paralysiert, unterbleiben die verstärkten Muskelanspannungen während der Tiefschlafphase, und es tritt kein Schnarchen auf.

Die Botulinumtoxine der Typen A, B, C1, D, E, F und G (BoNT/X) sind stark wirksame Neurotoxine, die eine über mehrere Wochen anhaltende Paralyse quergestreifter Muskeln auslösen (Ahnert-Hilger und Bigalke 1995). Die Ursache der Paralyse ist eine Hemmung der Azetylcholinfreisetzung aus den die Muskel versorgenden Nervenenden. Die Toxine sind Proteine und bestehen aus zwei miteinander kovalent verbundenen, ungleich großen Untereinheiten mit einer Gesamtmasse von M_{R} 150.000. Einige dieser Neurotoxine sind in einen Komplex (Gesamtmasse: M_{R} 900.000) eingebettet, der aus Hämagglutininen und nicht-toxischen Proteinen besteht (Inoue et al. 1995). Zur Muskelparalyse ist ausschließlich die Anwesenheit des Neurotoxins erforderlich, welches mit seiner größeren Kette, der C-terminalen Untereinheit des Toxins, an Rezeptoren bindet, die nur an Nervenzellen vorkommen. Über eine rezeptorvermittelte Endozytose wird das Toxin in Nervenzellen aufgenommen. Dort spalten die leichten Ketten, die N-terminalen Untereinheiten, zelleigene Proteine, die eine Schlüsselrolle bei der Verschmelzung transmitterenthaltender Vesikel mit der Plasmamembran einnehmen (Schiavo und Montecucco 1997). Als Folge der Spaltung unterbleibt die Fusion, und die Freisetzung des Transmitters ist blockiert: der Muskel kann nicht mehr kontrahiert werden. Am Sekretionsprozeß bzw. der Freisetzung sind mehrere der zelleigenen Proteine (Fusionsproteine) beteiligt, die in den Membranen sekretorischer Vesikel und/oder in der Plasmamembran vorhanden sind. Sie können auch im Zytosol vorkommen. Zu diesen Proteinen gehören SNAP 25, Synaptobrevin (VAMP) und Syntaxin bzw. deren Isoformen. Diese Proteine bilden den sogenannten Fusionskomplex, der die sekretorischen Vesikel an der Innenseite der Plasmamembran fixiert. Die Fixation geht der Membranverschmelzung voraus, die durch einen spannungsvermittelten Ca⁺⁺-Einstrom ausgelöst wird. Durch Inaktivierung auch nur eines der Fusionsproteine, etwa durch proteolytische Spaltung, wird die Bildung des Fusionskomplexes verhindert. Die Fusionsproteine sind die Zielmoleküle der leichten Ketten der oben genannten Neurotoxine. So spalten BoNT/B, D, F und G VAMP, während BoNT/A, C1 und E SNAP 25 inaktivieren und Syntaxin von BoNT/C1 zerschnitten wird. VAMP wird darüber hinaus noch von Tetanustoxin (TeNT) inaktiviert, einem Gift, welches ebenfalls zur Gruppe der Clostridien-Neurotoxine gehört (Ahnert-Hilger und Bigalke 1995).

BoNT/A wird bereits therapeutisch genutzt zur Behandlung verschiedener Formen lokaler, oft sehr schmerzhafter und den Patienten stark beeinträchtigenden Muskelverspannungen, z.B. von Torticollis spasmodicus, Blepharospasmus, diversen Spastizitäten usw. (Cardoso und Jankovic 1995). Das Toxin wird in den betroffenen Muskel injiziert. Nach wenigen Tagen ist der Muskel paralysiert. Der Patient wird schmerzfrei und kann wieder seinen täglichen Verpflichtungen nachkommen. Unerwünschte Wirkungen treten selten auf und sind, wie die erwünschten Wirkungen, voll reversibel.

### Beschreibung der Erfindung

Eine Beseitigung des Schnarchens ließe sich erreichen, wenn es gelänge, den erhöhten Tonus der Gaumenmuskulatur in der Tiefschlafphase zu unterdrücken. Da die Tonuserhöhung auf eine gesteigerte Azetylcholinfreisetzung zurückzuführen ist, kann die Blockade der Freisetzung die Muskulatur erschlaffen lassen und das Schnarchen beseitigen.

Die der Erfindung zugrundeliegende Aufgabe wird nun durch Verwendung von Clostridium-toxin oder einen Komplex dieses Toxins zur Herstellung eines intramuskulär zu verabreichenden Medikaments zur Unterdrückung von Schnarchgeräuschen gelöst.

So betrifft die Erfindung die Verwendung von hochreinem Clostridium-toxin BoNT/A.

So ist BoNT/A in geringer Dosierung in den betroffenen Muskel des weichen Gaumens, z.B. in den M. tensor veli palatini injizierbar. Mit der gleichen Injektionstechnik wird bereits die spasmodische Dysphonie behandelt, die ebenfalls auf einer Tonuserhöhung bestimmter Muskeln des weichen Gaumens beruht (Schönweiler et al. 1998).

Ferner betrifft die Erfindung die Verwendung von hochreinem Clostridium-toxin BoNT/B, BoNT/C1, BoNT/D, BoNT/E, BoNT/F und/oder BoNT/G.

Ferner betrifft die Erfindung die Verwendung von hochreinem Clostridium-toxin TeNT.

Ferner betrifft die Erfindung die Verwendung von
(i) einem Hybridprotein als Clostridium-toxin, bestehend aus einer leichten Untereinheit eines Clostridium-toxins der folgenden Gruppe und aus einer schweren Untereinheit eines anderen Clostridium-toxins derselben folgenden Gruppe, bestehend aus: BoNT/A, BoNT/B, BoNT/C1, BoNT/D, BoNT/E, BoNT/F, BoNT/G und TeNT; oder
(ii) einem Gemisch von Hybridproteinen gemäß (i). Ferner betrifft die Erfindung die Verwendung von einem Komplex, umfassend

(i) ein Clostridium-toxin oder ein Hybridprotein und
(ii) ein oder mehrere therapeutisch verträgliche Hämagglutinine und/oder ein oder mehrere pharmazeutisch verträgliche nicht-toxische Proteine.

Ferner betrifft die Erfindung die Verwendung von einem Komplex vom Wildtyp.

Ferner betrifft die Erfindung die Verwendung von einem Clostridium-toxin, bei dem es sich um ein rekombinantes Protein handelt.

Ferner betrifft die Erfindung die Verwendung von einem Clostridium-toxin, bei dem es sich um ein lyophilisiertes Produkt handelt.

Ferner betrifft die Erfindung die Verwendung von einem Clostridium-toxin, bei dem es sich um line wässerige Lösung, insbesondere um line wässerige Injektionslösung, handelt.

Ferner betrifft die Erfindung die Verwendung von Clostridium-toxin oder seinem Komplex in physiologischer Kochsalzlösung.

Schließlich betrifft die Erfindung die Verwendung von Liposomen als Träger für das Clostridium-toxin oder seinen Komplex.

Die Applikation reinen Neurotoxins ist gegenüber der Injektion des Komplexes bevorzugt, denn nur das Neurotoxin trägt das Wirkprinzip. Da das Neurotoxin eine kleinere Molekülmasse als der Komplex besitzt, verteilt es sich durch Diffusion schnell im Muskelgewebe, bindet an Rezeptoren und hemmt die Azetylcholinfreisetzung, nachdem es in das Nervenende aufgenommen wurde. Die anderen körperfremden Proteine hätten keine Eigenwirkung bezüglich der Muskelparalyse. Sie trügen aber zur Stimulation des Immunsystemes bei, weil sie als Immunadjuvanzien wirken und die Immunreaktionen verstärken. Die stärkere Immunantwort ist erwünscht bei Impfungen. Im Falle des Schnarchtherapeutikums könnte jedoch eine Immunreaktion zu einer unerwünschten Bildung von Antikörpern führen, die das Toxin bei einer erneuten Gabe neutralisieren würden, bevor es wirken könnte.

Alle Körperöffnungen, und so auch der Nasen-Rachen Bereich, sind reich an lymphatischem Gewebe, das die Eintrittspforten vor schädigenden Substanzen schützt. Kommt es zu Verletzungen und Eindringen körperfremder Substanzen in diesem Bereich, werden Makrophagen angelockt, die die Fremdstoffe aufnehmen, verdauen und Fragmente der Fremdstoffe mit zelleigenen Proteinen auf ihrer Zelloberfläche exprimieren. In der Milz und anderen lymphatischen Geweben erkennen Lymphozyten die Fragmente und bilden Immunglobuline, die frei im Gewebe liegende Fremdstoffe binden und diese neutralisieren.

Das Ausmaß der Anlockung der Makrophagen hängt unter anderem von der Konzentration an Fremdstoffen und von der Verfügbarkeit von Makrophagen ab. Die Verfügbarkeit von Makrophagen kann nicht beeinflußt werden, und der Gaumenbereich ist reich an diesen Zellen. Um die Wahrscheinlichkeit einer Immunantwort gering zu halten, muß die Masse von Fremdproteinen, i.e. Neurotoxin und Hämagglutinine, so gering wie möglich sein, denn Makrophagen, die in großer Anzahl von nicht-wirksamen Fremdstoffen (Immunadjuvanzien) angelockt werden, nehmen natürlich auch das im Gewebe liegende Neurotoxin auf. Die Masse des Neurotoxins läßt sich durch ein BoNT mit hoher biologischer Aktivität auf eine gerade noch wirksame Dosis reduzieren. Da die Begleitproteine nicht zur erwünschten Wirkung beitragen, können sie erfindungsgemäß entfernt werden.

### Beispiel 1:

Eine indirekte Injektion unter Videolaryngoskopie wurde an drei gesunden, aufrecht sitzenden Männern vorgenommen. Dreißig Minuten vor der. Toxininjektion wurde Atropinsulfat (0,5 mg) subcutan appliziert, um die Speichelsekretion zu unterdrücken. Der Oropharynx, der Mesopharynx und der Larynx wurden oberflächlich mit Tetracainhydrochlorid (1 %) anästhesiert, dem Adrenalinhydrochlorid (1,2 mg) zugesetzt war, um eine lokale Gefäßkonstriktion zu erzeugen. Lyophilisiertes, gereinigtes BoNT/A (i.e. frei von körperfremden Begleitproteinen; BioteCon, Berlin) wurde in physiologischer Kochsalzlösung p.i. gelöst (300 pg/ml). Die Injektion erfolgte mit einer gebogenen Injektionskanüle unter Videokontrolle. Die Männer wurden aufgefordert, beim Einstechen der Kanüle normal zu atmen. An drei Stellen des M. tensor veli palatini wurden bei dem ersten Probanden jeweils 100 µl, bei dem zweiten 200 und bei dem dritten 300 µl injiziert. Die Dosis des Toxins lag somit bei 50 - 150 pg/Gaumen, das entspricht 8 - 25 U. Die Gaumenmuskulatur erschlaffte daraufhin innerhalb von 3 - 5 Tagen. Die Wirkung des Toxins hielt für 3 - 4 Monate an. Nach dieser Zeitspanne traten, zuerst leise, dann immer lauter werdend, die Schnarchgeräusche wieder auf.

Sowohl subjektiv (Befragung des Partners) als auch objektivierbar durch Messungen wurde eine Reduktion des Schnarchens festgestellt. Der Grad der Erschlaffung der Gaumenmuskel wurde durch eine Frequenzanalyse der Schnarchgeräusche ermittelt. Abhängig von der Toxindosis nahm die Frequenz der Schnarchgeräusche ab. Weiterhin nahm die Lautstärke des Schnarchens ab, welches auf einer Vergrößerung des Querschnittes der oberen Luftwege beruhte. Durch die Vergrößerung nimmt die Strömungsgeschwindigkeit der Atemluft ab, wodurch die Amplitude der Schwingung des weichen Gaumens geringer wurde. Schluck- oder Sprechstörungen traten nicht auf.

### Beispiel 2:

Wie unter Beispiel 1 beschrieben, wurde einer Versuchsperson BoNT/B in den M. tensor veli palatini injiziert. Die Gesamtdosis betrug 15 pg, die auf vier Injektionspunkte verteilt wurden. Das Injektionsvolumen betrug 1 ml. Die Gaumenmuskulatur erschlaffte daraufhin innerhalb von 3 - 5 Tagen. Die Wirkung des Toxins hielt für ca. 6 Wochen an. Nach dieser Zeitspanne traten, zuerst leise, dann immer lauter werdend, die Schnarchgeräusche wieder auf.

Sowohl subjektiv (Befragung des Partners) als auch objektivierbar durch Messungen wurde eine Reduktion des Schnarchens festgestellt. Der Grad der Erschlaffung der Gaumenmuskel wurde, wie schon unter Beispiel 1 beschrieben, objektiviert. Schluckoder Sprechstörungen traten auch nach Injektion dieses Subtyps nicht auf.

### Beispiel 3:

Wie unter Beispiel 1 beschrieben, wurde einer Versuchsperson BoNT/C1 in den M. tensor veli palatini injiziert. Diese Versuchsperson wurde seit mehreren Jahren wegen eines torticollis spasmodicus mit BoNT/A-Komplex (BOTOX®, Merz GmbH & Co. KG) behandelt. Vor einem Jahr (1997) konnte keine Paralyse der Halsmuskulatur trotz einer Verdopplung der Dosis von 150 U auf 300 U mehr ausgelöst werden. Daraufhin wurden bei diesem Patienten mit Hilfe eines isolierten Nerv-Muskelpräparates neutralisierende Antikörper nachgewiesen (Göschel et al. 1997). Um auch diesen Patienten von seinem Schnarchen zu befreien, bekam er BoNT/C1. Die Gesamtdosis betrug 50 pg, die auf vier Injektionspunkte verteilt wurden. Die erhöhte Dosis wurde benötigt, da Typ C1 eine geringere spezifische Toxizität besitzt als die Typen A und B. Das Injektionsvolumen betrug 1 ml. Die Gaumenmuskulatur erschlaffte daraufhin innerhalb von 3 - 5 Tagen. Die Wirkung des Toxins hielt für ca. 14 Wochen an. Nach dieser Zeitspanne traten, zuerst leise, dann immer lauter werdend, die Schnarchgeräusche wieder auf.

Sowohl subjektiv (Befragung des Partners) als auch objektivierbar durch Messungen wurde eine Reduktion des Schnarchens festgestellt. Der Grad der Erschlaffung der Gaumenmuskel wurde, wie schon unter Beispiel 1 beschrieben, objektiviert. Schluckoder Sprechstörungen traten auch nach Injektion dieses Subtyps nicht auf.

### Referenzen

Ahnert-Hilger G, Bigalke H: Molecular aspects of tetanus and botulinum neurotoxin poisoning. Progress Neurobiol 46: 83-96, 1995.

Cardoso F, Jankovic J: Clinical use of botulinum neurotoxins. Top Microbiol Immunol 195: 123-141, 1995.

Göschel H., Wohlfarth K., Frevert J. et al: Botulinum A Toxin Therapy: Neutralizing ans Nonneutralizing Antibodies - Therapeutic Consequences. Experimental Neurology 14: 96-102, 1997

He J, Kryger MH, Zorich FJ, et al: Mortality and apnea index in obstructive sleep apnea. Chest 94: 9-14. 1988.

Hoch CC, Reynolds CF III, Kupfer DJ, et al: Sleep disordered breathing in normal and pathologic aging. J Clin Psychol 47: 499-503, 1986.

Hoffstein V, Mateika JH, Mateika S: Snoring and sleep architecture. Am Rev Respir Dis 143: 92-96, 1991

Kleitman N: Sleep and wakefulness. Chicago, The University of Chicago Press, 1963.

Koskenvuo M, Kaprio J, Partinen M, et al: Snoring as a risk factor for hypertension and angina pectoris. Lancet i: 893-896, 1985.

Koskenvuo M, Kaprio J, Telakivi T, et al: Snoring as a risk factor for ischaemic heart disease and stroke in men. Br Med J 294: 16-19, 1987.

Lugaresi E., Cirignotta F., Montagna P. et al: Snoting: Pathogenic, Clinical, and Therapeutic Aspects. In Abnormal Sleep. 621-629, 1994

Lugaresi E, Mondini S, Zucconi M, et al: Staging of heavy snorers disease. A proposal. Bull Eur Physiopathol Respir 19:590-594, 1983.

Schiavo G, Montecucco C: The structure and mode of action of botulinum and tetanus toxin. Clostridia: Mol Biol Pathog , 295-321,1997.

Schönweiler R, Wohlfahrt K, Dengler R, Ptok M: Supraglottal injection of botulinum toxin type A in adductor type spasmodic dysphonia with both intrinsic and extrinsic hyperfunction. Laryngoscope 108: 55-63, 1998.

Waller PC, Bhopal RS: Is snoring a cause of vascular disease? An epidemiological review. Lancet i: 143-146, 1989.

## Patentansprüche

1. Verwendung von hochreinem Clostridium-toxin BoNT/A zur Herstellung eines intramuskulär zu verabreichenden Medikaments zur Unterdrückung von Schnarchgeräuschen.

2. Verwendung von hochreinem Clostridium-toxin BoNT/B, BoNT/C1, BoNT/D, BoNT/E, BoNT/F und/oder BoNT/G zur Herstellung eines intramuskulär zu verabreichenden Medikaments zur Unterdrückung von Schnarchgeräuschen.

3. Verwendung von hochreinem Clostridium-toxin TeNT zur Herstellung eines intramuskulär zu verabreichenden Medikaments zur Unterdrückung von Schnarchgeräuschen.

4. Verwendung (i) eines Hybridproteins als Clostridium-toxin, bestehend aus einer leichten Untereinheit eines Clostridium-toxins der folgenden Gruppe und aus einer schweren Untereinheit eines anderen Clostridium-toxins derselben folgenden Gruppe, bestehend aus: BoNT/A, BoNT/B, BoNT/C1, BoNT/D, BoNT/E, BoNT/F, BoNT/G und TeNT; oder (ii) eines Gemisches von Hybridproteinen gemäß (i) zur Herstellung eines intramuskulär zu verabreichenden Medikaments zur Unterdrückung von Schnarchgeräuschen.

5. Verwendung eines Komplexes umfassend ein Clostridium-toxin oder ein Hybridprotein und ein oder mehrere therapeutisch verträgliche Hämagglutinine und/oder ein oder mehrere pharmazeutisch verträgliche nicht-toxische Proteine zur Herstellung eines intramuskulär zu verabreichenden Medikaments zur Unterdrückung von Schnarchgeräuschen.

6. Verwendung nach Anspruch 5, **dadurch gekennzeichnet, dass** ein Komplex vom Wildtyp verwendet wird.

7. Verwendung nach einem der vorhergehenden Ansprüche, **dadurch gekennzeichnet, dass** es sich bei dem Clostridium-toxin um ein rekombinantes Protein handelt.

8. Verwendung nach einem der vorhergehenden Ansprüche, **dadurch gekennzeichnet, dass** es sich bei dem Clostridium-toxin um ein lyophilisiertes Produkt handelt.

9. Verwendung nach einem der Ansprüche 1 bis 7, **dadurch gekennzeichnet, dass** es als wässerige Lösung vorliegt, insbesondere als wässerige Injektionslösung.

10. Verwendung nach Anspruch 9, **dadurch gekennzeichnet, dass** das Clostridium-toxin oder sein Komplex in physiologischer Kochsalzlösung vorliegen.

11. Verwendung nach einem der Ansprüche 1 bis 7, **dadurch gekennzeichnet, dass** Liposomen als Träger für das Clostridium-toxin oder seinen Komplex verwendet werden.

## Claims

1. Use of high-purity Clostridium toxin BoNT/A for manufacturing a therapeutic agent, to be administered intramuscularly, for suppressing snoring noises.

2. Use of high-purity Clostridium toxin BoNT/B, BoNT/C1, BoNT/D, BoNT/E, BoNT/F and/or BoNT/G for manufacturing a therapeutic agent, to be administered intramuscularly, for suppressing snoring noises.

3. Use of high-purity Clostridium toxin TeNT for manufacturing a therapeutic agent, to be administered intramuscularly, for suppressing snoring noises.

4. Use of a hybrid protein as Clostridium toxin, comprised of a light subunit of a Clostridium toxin of the following group and of a heavy subunit of a different Clostridium toxin of the same following group, comprised of: BoNT/A, BoNT/B, BoNT/C1, BoNT/D, BoNT/ E, BoNT/F, BoNT/G and TeNT; or a mixture of hybrid proteins according to (i) for manufacturing a therapeutic agent, to be administered intramuscularly, for suppressing snoring noises.

5. Use of a complex comprising a Clostridium toxin or a hybrid protein and one or more therapeutically well-tolerated hemagglutinins and/or one or more pharmaceutically well-tolerated non-toxic proteins for the manufacture of a therapeutic agent, to be administered intramuscularly, for suppressing snoring noises.

6. Use according to claim 5, **characterized in that** a complex of the wild type is employed.

7. Use according to one of the preceding claims, **characterized in that** the Clostridium toxin is a recombinant protein.

8. Use according to one of the preceding claims, **characterized in that** the Clostridium toxin is a lyophilized product.

9. Use according to one of the claims 1 to 7, **characterized in that** it is present as an aqueous solution, in particular, an aqueous injection solution.

10. Use according to claim 9, **characterized in that** the Clostridium toxin or its complex is present as a physiological saline solution.

11. Use according to one of the claims 1 to 7, **characterized by** liposomes as carriers for the Clostridium toxin or its complex.

## Revendications

1. Utilisation de la toxine BoNT/A de Clostridium de pureté élevée pour la fabrication d'un médicament pour administration intramusculaire pour la diminution du ronflement.

2. Utilisation de la toxine BoNT/B, BoNT/C₁, BoNT/D, BoNT/E, BoNT/F et/ou BoNT/G de Clostridium de pureté élevée pour la fabrication d'un médicament pour administration intramusculaire pour la diminution du ronflement.

3. Utilisation de la toxine TeNT de Clostridium de pureté élevée pour la fabrication d'un médicament pour administration intramusculaire pour la diminution du ronflement.

4. Utilisation (i) en tant que toxine de Clostridium d'une protéine hybride consistant en une sous-unité légère d'une toxine de Clostridium du groupe suivant et d'une sous-unité lourde d'une autre toxine de Clostridium du même groupe consistant en : BoNT/A, BoNT/B, BoNT/C₁, BoNT/D, BoNT/E, BoNT/F, BoNT/G et TeNT ; ou (ii) d'un mélange de protéines hybrides selon (i) pour la fabrication d'un médicament pour administration intramusculaire pour la diminution du ronflement.

5. Utilisation d'un complexe comprenant une toxine de Clostridium ou d'une protéine hybride et d'une ou plusieurs hémaglutinines acceptables sur le plan thérapeutique et/ou d'une ou plusieurs protéines non toxiques acceptables sur le plan pharmaceutique pour la fabrication d'un médicament pour administration intramusculaire pour la diminution du ronflement.

6. Utilisation selon la revendication 5, **caractérisée en ce que** l'on utilise un complexe de type sauvage.

7. Utilisation selon l'une des revendications précédentes, **caractérisée en ce que** il s'agit pour la toxine de Clostridium d'une protéine recombinante.

8. Utilisation selon l'une des revendications précédentes, **caractérisée en ce que** il s'agit pour la toxine de Clostridium d'un produit lyophilisé.

9. Utilisation selon l'une des revendications 1 à 7, **caractérisée en ce que** la toxine de Clostridium ou son complexe se présente en solution aqueuse, en particulier sous forme d'une solution aqueuse pour injections.

10. Utilisation selon la revendication 9, **caractérisée en ce** la toxine de Clostridium ou son complexe se présente en solution saline physiologique.

11. Utilisation selon l'une des revendications 1 à 7, **caractérisée en ce que** l'on utilise des liposomes en tant que support pour la toxine de Clostridium ou son complexe.
